# EUROPEAN PATENT APPLICATION

(11) **EP 2 940 102 A1**
(43) Date of publication of application: **04.11.2015**
(21) Application number: 14166772.5
(22) Date of filing: 01.05.2014
(51) Int. Cl.: C10G 2/00, B01J 19/00, C07C 1/04

(54) **A method for start-up and operation of a Fischer-Tropsch reactor**

(71) Applicant: SHELL INTERNATIONAL RESEARCH MAATSCHAPPIJ B.V., 2596 HR Den Haag (NL)
(72) Inventor: Bezemer, Gerrit Leendert, 1031HW Amsterdam (NL); Mesters, Carolus Matthias Anna Maria, Sugar Land, TX 77498 (US)
(74) Representative: Matthezing, Robert Maarten

(57) **Abstract**

The invention relates to a method for start-up and operation of a Fischer-Tropsch reactor comprising the following sequential steps:
(α) providing a reactor with a fixed bed of reduced Fischer-Tropsch catalyst comprising cobalt as catalytically active metal wherein the catalyst has an initial intrinsic activity;
(b) deactivating part of the catalyst by providing part of the catalyst with a form of carbon;
(c) supplying a gaseous feed stream comprising carbon monoxide and hydrogen in a ratio in the range of from 0.5:1 to 2:1 to the reactor and converting carbon monoxide and hydrogen supplied with the gaseous feed stream to the reactor into hydrocarbons at a reaction temperature and reaction pressure.

## Description

### Field of the invention

The present invention relates to a method for start-up of a Fischer-Tropsch reactor containing a Fischer-Tropsch catalyst, wherein at least during start-up of the reactor the catalyst has a decreased intrinsic activity.

The catalyst is suitable for use in producing normally gaseous, normally liquid and optionally solid hydrocarbons from synthesis gas generally provided from a hydrocarbonaceous feed, in a Fischer-Tropsch process. In the current specification such a catalyst is referred to as a Fischer-Tropsch catalyst.

### Background to the invention

The Fischer-Tropsch process can be used for the conversion of synthesis gas into liquid and/or solid hydrocarbons. The synthesis gas may be obtained from hydrocarbonaceous feedstock in a process wherein the feedstock, e.g. natural gas, associated gas and/or coal-bed methane, heavy and/or residual oil fractions, coal, biomass, is converted in a first step into a mixture of hydrogen and carbon monoxide. This mixture is often referred to as synthesis gas or syngas. The synthesis gas is then fed into a reactor where it is converted in one or more steps over a suitable catalyst at elevated temperature and pressure into paraffinic compounds and water in the actual Fischer-Tropsch process. The obtained paraffinic compounds range from methane to high molecular weight modules. The obtained high molecular weight modules can comprise up to 200 carbon atoms, or, under particular circumstances, even more carbon atoms. Numerous types of reactor systems have been developed for carrying out the Fischer-Tropsch reaction. For example, Fischer-Tropsch reactor systems include fixed bed reactors, especially multi-tubular fixed bed reactors, fluidised bed reactors, such as entrained fluidised bed reactors and fixed fluidised bed reactors, and slurry bed reactors such as three-phase slurry bubble columns and ebulated bed reactors.

Catalysts used in the Fischer-Tropsch synthesis often comprise a carrier-based support material and one or more metals from Groups 8-10 of the Periodic Table of Elements, especially from the cobalt or iron groups, optionally in combination with one or more metal oxides and/or metals as promoters selected from zirconium, titanium, chromium, vanadium and manganese, especially manganese. Such catalysts are known in the art and have been described for example, in the specifications of WO 9700231A and US 4595703.

One of the limitations of a Fischer-Tropsch process is that the activity of the catalyst will, due to a number of factors, decreases over time. The activity of the catalyst is decreased as compared to its initial catalytic activity. The initial activity of the catalyst can be its activity when freshly prepared. A catalyst that shows a decreased activity after use in a Fischer-Tropsch process is sometimes referred to as deactivated catalyst, even though it usually still shows activity. Sometimes such a catalyst is referred to as a deteriorated catalyst. Sometimes it is possible to regenerate the catalyst. This may be performed, for example, with one or more oxidation and/or reduction steps.

After regeneration, catalysts often show an activity that is lower than the activity of freshly prepared catalysts. Especially after multiple regenerations, it often proofs hard to regain an activity level comparable to the activity of freshly prepared catalysts. In order to be able to use a catalyst for a long time, it thus may be desirable to start a Fischer-Tropsch process with a fresh catalyst that has a relatively high activity.

The use of fresh or rejuvenated catalysts with a relatively high initial activity may have disadvantages. This may especially be the case when the amount of catalyst used in a reactor tube is fixed after loading of the catalyst in the reactor tube. One example of a reactor tube filled with a fixed amount of catalyst is a reactor tube filled with a packed bed of catalyst particles.

In a Fischer-Tropsch process with a catalyst with a relatively high initial activity, the activity of the catalyst is especially high at the start of the process. And, due to the high activity of the catalyst, a lot of water is produced in the Fischer-Tropsch hydrocarbon synthesis, resulting in a high relative humidity at the start of the Fischer-Tropsch process. During Fischer-Tropsch synthesis the relative humidity in a reactor tube may increase to such a level that it accelerates the deactivation of the catalyst during use. During start-up of a Fischer-Tropsch reactor with a very active catalyst, the reaction temperature is typically kept at a relatively low value, e.g. below 200 °C, in order to avoid a too high product yield and accompanying high temperature rise due to the exothermic reaction. Without wishing to be bound to any theory, it is believed that especially the combination of relatively low temperature and a relatively high yield results in a high relative humidity in the reactor and therewith in undesired irreversible catalyst deactivation.

Therefore, especially in the start-up phase of a Fischer Tropsch reactor with a catalyst with a relatively high activity, the deactivation of the catalyst takes place at a relatively high rate. Deactivation due to relative humidity proofed to be difficult to reverse. The high initial activity in such a case is thus quickly lost, whereas regeneration procedures have only a limited effect.

### Summary of the invention

It is an object of the present invention to provide an improved Fischer-Tropsch process in which a cobalt catalyst is used that has a relatively high initial activity. Especially the start-up of a Fischer-Tropsch reactor provided with fresh catalyst is improved. Preferably, also the start-up of a Fischer-Tropsch reactor after a regeneration step is improved.

It has now been found that the conditions of relative humidity and therewith of increased catalyst deactivation at the start-up of a Fischer-Tropsch reactor that has been loaded with fresh or rejuvenated catalyst can be avoided by operating a Fischer-Tropsch reactor such that part of the catalyst in a fixed bed of catalyst is inactive or less active during start-up and in the initial stages of operation of the Fischer-Tropsch reactor. In particular it has been found that by proving a fixed bed of catalyst wherein a form of carbon, preferably a form of carbon selected from the group consisting of carbide, graphite carbon or coke, is deposited on the catalyst, the overall catalyst activity in the reactor is decreased and as a result the reaction temperature can be increased in order to achieve a desired set reactor productivity. Such conditions of higher temperature and decreased overall catalyst activity result in a lower relative humidity at the start-up of the reactor and therewith in less catalyst deactivation.

Accordingly, the present invention relates to a method for start-up and operation of a Fischer-Tropsch reactor comprising the following sequential steps:
(a) providing a reactor with a fixed bed of reduced Fischer-Tropsch catalyst comprising cobalt as catalytically active metal wherein the catalyst has an initial intrinsic activity;
(b) deactivating part of the catalyst by providing part of the catalyst with a form of carbon;
(c) supplying a gaseous feed stream comprising carbon monoxide and hydrogen in a ratio in the range of from 0.5:1 to 2:1 to the reactor and converting carbon monoxide and hydrogen supplied with the gaseous feed stream to the reactor into hydrocarbons at a reaction temperature and reaction pressure.

An important advantage of the method of the invention is that a higher reaction temperature is allowed in the start-up phase and the initial phase of the operation of the reactor, compared to the initial reaction temperature in a reactor wherein no carbon is deposited on part of the catalyst in the catalyst bed, resulting in a lower relative humidity. It has further been found that the selectivity for C5+ hydrocarbons is not importantly affected by the higher reaction temperature during start-up and initial phase of operation of the reactor.

Another advantage of the method according to the invention is that, compared to start-up methods wherein a relatively low initial temperature is used to avoid a too high temperature of the reactor at or shortly after start-up, heat recovery from the process is improved, since steam of a higher quality can be produced.

### Detailed description of the invention

The method according to the invention is a method for start-up and operation of a Fischer-Tropsch reactor. The method comprises a first step of providing a reactor with a fixed bed of reduced Fischer-Tropsch catalyst. The catalyst comprises cobalt as catalytically active metal.

The catalyst may be a fresh catalyst or a rejuvenated catalyst. Reference herein to a fresh catalyst is to a freshly prepared catalyst that has not been subjected to a Fischer-Tropsch process. Reference herein to a rejuvenated catalyst is to a regenerated catalyst of which the initial activity has been at least partially restored, typically by means of several reduction and/or oxidation steps. The catalyst is preferably a fresh catalyst, since in particular fresh catalysts have a very high initial activity.

The catalyst comprises cobalt as catalytically active metal. Fischer-Tropsch catalysts comprising cobalt as catalytically active metal are known in the art. Any suitable cobalt-comprising Fischer-Tropsch catalysts known in the art may be used. Typically such catalyst comprises cobalt on a carrier-based support material, optionally in combination with one or more metal oxides and/or metals as promoters selected from zirconium, titanium, chromium, vanadium and manganese, especially manganese. A most suitable catalyst comprises cobalt as the catalytically active metal and titania as carrier material.

The catalyst may further comprise one or more promoters. One or more metals or metal oxides may be present as promoters, more particularly one or more d-metals or d-metal oxides. Suitable metal oxide promoters may be selected from Groups 2-7 of the Periodic Table of Elements, or the actinides and lanthanides. In particular, oxides of magnesium, calcium, strontium, barium, scandium, yttrium, lanthanum, cerium, titanium, zirconium, hafnium, thorium, uranium, vanadium, chromium and manganese are most suitable promoters. Suitable metal promoters may be selected from Groups 7-10 of the Periodic Table of Elements. Manganese, iron, rhenium and Group 8-10 noble metals are particularly suitable as promoters, and are preferably provided in the form of a salt or hydroxide.

The promoter, if present in the catalyst, is typically present in an amount of from 0.001 to 100 parts by weight per 100 parts by weight of carrier material, preferably 0.05 to 20, more preferably 0.1 to 15. It will however be appreciated that the optimum amount of promoter may vary for the respective elements which act as promoter.

A most suitable catalyst comprises cobalt as the catalytically active metal and zirconium as a promoter. Another most suitable catalyst comprises cobalt as the catalytically active metal and manganese and/or vanadium as a promoter. If the catalyst comprises cobalt as the catalytically active metal and manganese and/or vanadium as promoter, the cobalt: (manganese + vanadium) atomic ratio is advantageously at least 12:1.

It has been found that the method according to the invention is particularly suitable for start-up and operation of a Fischer-Tropsch reactor containing a catalyst without a noble metal as promoter. Therefore, the catalyst preferably does not comprise a noble metal.

References to "Groups" and the Periodic Table as used herein relate to the new IUPAC version of the Periodic Table of Elements such as that described in the 87th Edition of the Handbook of Chemistry and Physics (CRC Press).

In step (a) a reactor is provided with a fixed bed of the catalyst, preferably with a fixed bed of catalyst particles, more preferably in a multi-tubular fixed bed reactor configuration.

The reactor is provided with a fixed bed of reduced catalyst in step (a). In a reduced catalyst the cobalt is essentially in its metallic state. The reactor may be provided with a fixed bed of reduced catalyst by reducing a fixed bed of catalyst precursor in-situ, i.e. in the same reactor wherein the Fischer-Tropsch hydrocarbon synthesis will take place, or by loading the reactor with a reduced catalyst that has for example be prepared by reducing a catalyst precursor in a separate vessel or reactor prior to loading the reduced catalyst in the reactor. Preferably the reactor is provided with a fixed bed of reduced catalyst by reducing a fixed bed of catalyst precursor in-situ. Reference herein to a catalyst precursor is to a precursor that can be converted into a catalytically active catalyst by subjecting the precursor to reduction, usually by subjecting the precursor to hydrogen or a hydrogen-containing gas under reducing conditions.

Such catalyst precursor reduction is well-known in the art. The catalyst precursor may be reduced by any known manner under conventional conditions. For example, the catalyst precursor may be activated by contacting it with hydrogen or a hydrogen-containing gas, typically at temperatures of about 200° to 350 °C. During reduction the pressure may be in the range of between 0 and 100 bar (absolute), preferably in the range of between 1 and 60 bar, more preferably in the range of between 1 and 10 bar, even more preferably in the range between 5 and 10 bar.

The reduced catalyst provided in step (a) has an initial intrinsic activity.

Once the reactor has been provided with a fixed bed of reduced catalyst in step (a), typically by first providing a bed of catalyst precursor and then in-situ reducing the catalyst precursor, part of the catalyst in the fixed bed is deactivated by provided part of the catalyst with a form of carbon, preferably a form of carbon selected from the group consisting of carbide, graphite, and coke, in order to decrease the overall intrinsic catalyst activity over the entire fixed bed. Preferably, part of the catalyst is provided with carbide by converting part of the metallic cobalt in the reduced catalyst into cobalt carbide or part of the catalyst is provided with graphite, suitably by first forming cobalt carbide and then thermally converting the cobalt carbide into graphite.

Preferably, the deactivation in step (b) is such that more catalyst is deactivated in an upstream part of the fixed bed of catalyst than in a downstream part of the fixed bed of catalyst. Preferably therefore, the fixed bed consists, after deactivation step (b), of an upstream part and a downstream part upstream part, wherein a larger part of catalyst is deactivated in the upstream part than in the downstream part. Reference herein to upstream and downstream is with respect to the direction of flow of the gaseous feed stream during normal operation of the reactor.

Preferably the catalyst is deactivated such in step (b) that the catalyst in the upstream part of the fixed bed has an intrinsic activity that is less than 70% of the initial intrinsic activity and the catalyst in the downstream part of the fixed bed has an intrinsic activity that is at least 70% of the initial intrinsic activity.

Preferably, the upstream part of the fixed bed has a length in the range of from 10 to 90% of the total length of the fixed bed, more preferably of from 15 to 50% of the total length of the fixed bed.

Once part of the catalyst in the fixed bed has been provided with a form of carbon in step (b), Fischer-Tropsch hydrocarbon synthesis is started in step (c) by supplying a gaseous feed stream comprising carbon monoxide and hydrogen in a volume ratio in the range of from 0.5:1 to 2:1 to the reactor and converting carbon monoxide and hydrogen supplied with the gaseous feed stream to the reactor into hydrocarbons at a reaction temperature and a reaction pressure.

The conversion in step (c) may be carried out at any suitable reaction pressure. Preferably, the reaction temperature is chosen such that the reactor operates at a set reactor productivity, preferably a reactor productivity in the range of from 75 to 500 grams hydrocarbons per liter of catalyst per hour, more preferably in the range of from 100 to 350 grams hydrocarbons per liter of catalyst per hour.

Reference herein to reactor productivity is to space time yield of the reactor, i.e. the amount of hydrocarbons produced per volume of catalyst per hour (g/l.h).

Reference herein to activity of the catalyst is to the intrinsic activity of the catalyst. It is thus a property of the catalyst and not dependent on the actual reaction conditions applied.

Reference herein to the reaction temperature is to the temperature of coolant, typically cooling water, surrounding the fixed bed of catalyst.

Reference herein to the gaseous feed stream to the reactor is to the combined feed stream to the reactor including any gaseous recycle stream.

It will be appreciated that for a catalyst with a given activity, and for given conditions such as reaction pressure, gas hourly space velocity and reactor configuration, reaction temperature and yield (reactor productivity) are directly related. If the reactor productivity is to be set at a certain value, reaction temperature is a resulting parameter and *vice versa.*

Since in the method according to the invention part of the catalyst in the fixed bed is provided with a form of carbon and therefore inactive, the overall catalyst activity in the reactor is decreased compared to the initial overall catalyst activity, i.e. prior to deactivation step (b). The overall catalyst activity is mainly determined by the relative amount of catalyst provided with a form of carbon in the reactor. Due to this lower activity, the initial reaction temperature needed to achieve a desired set value for the reactor productivity is sufficiently high to avoid a combination of low reaction temperature and high yield resulting in a high relative humidity in the reactor that might be detrimental for the stability of the catalyst, i.e. would result in irreversible and/or rapid catalyst deactivation.

The conversion of carbon monoxide and hydrogen into hydrocarbons in step (c) may be carried out at any reaction pressure and gas hourly space velocity known to be suitable for Fischer-Tropsch hydrocarbon synthesis. Preferably, the reaction pressure is in the range of from 10 to 100 bar (absolute), more preferably of from 20 to 80 bar (absolute). The gas hourly space velocity is preferably in the range of from 500 to 25,000 h⁻¹, more preferably of from 900 to 15,000 h⁻¹, even more preferably of from 1,300 to 8,000 h⁻¹. Preferably, the reaction pressure and the gas hourly space velocity are kept constant in step (c).

The method according to the invention may be applied for the start-up and further operation of a Fischer-Tropsch reactor with a fresh catalyst or with a regenerated catalyst. The method according to the invention is particularly suitable for a Fischer-Tropsch reactor containing a fresh catalyst.

Preferably in step (c), carbon monoxide and hydrogen are converted into hydrocarbons at a set reactor productivity and the reaction temperature is initially at least 200 °C, and the set reactor productivity is maintained by adjusting the reaction temperature. More preferably, the reaction temperature is initially set at a value of at least 203 °C, even more preferably at least 205 °C, still more preferably at a value in the range of from 205 to 220 °C, still more preferably of from 210 to 215 °C.

Reference herein to maintaining the reactor productivity is to maintaining the reactor productivity within 10% of the set value, i.e. in a range of from 90 to 110% of the set value.

It will be appreciated that for a catalyst with a given activity, the value at which the initial reaction temperature is set is chosen such that under the prevailing other conditions, such as reaction pressure and space velocity, and for the given reactor configuration, the resulting yield and therewith water production is such that a relative humidity might be detrimental for the stability of the catalyst, i.e. would result in irreversible and/or rapid catalyst deactivation, is avoided. The initial overall catalyst activity is mainly determined by the relative amount of catalyst provided with carbon in the reactor

During operation of a Fischer-Tropsch process, the catalyst is typically gradually deactivated. In order to compensate for any changes in overall catalyst activity, the reaction temperature is preferably adjusted during operation of the reactor, to maintain the reactor productivity at its set value.

Step (b), i.e. provision of part of the catalyst in the fixed bed with a form of carbon, may be done in several ways.

In one preferred embodiment of the invention, part of the catalyst is provided with carbide by contacting the reduced catalyst with a gaseous stream comprising carbon monoxide, wherein the gaseous stream comprises less than 10 vol% of hydrogen, at a temperature in the range of from 150 to 290 °C and preferably a pressure in the range of from 1 to 80 bar (absolute), to convert at least part of the cobalt in the catalyst into cobalt carbide.

Preferably, the gaseous stream comprises less than 5 vol% hydrogen, more preferably less than 1 vol% hydrogen, even more preferably less than 0.5 vol%, still more preferably less than 0.1 vol%.

The gaseous stream comprising carbon monoxide may comprise an inert gas such as nitrogen or low-sulphur natural gas. The gaseous stream comprising carbon monoxide may comprise up to 99.5 vol% of inert gas. For example, when the gas flow rate is high, the inert content of the gaseous stream comprising carbon monoxide may be low or high, but preferably is high. When the gas flow rate is high, it is easier to control the reaction when the inert content is high and the carbon monoxide content is low. When the gas flow rate is low, the inert content of the gaseous stream comprising carbon monoxide may be high or low, but preferably is low or even zero.

The gaseous stream comprising carbon monoxide may for example be contacted with the fixed bed in an amount of 0.5 Nl/l.h up to 550 Nl/l.h. The choice of gas flow rate may, among others, depend on the type of fixed bed, the size of the fixed bed, and whether or not multiple tubes with a fixed bed inside are treated at the same time. The gaseous stream comprising carbon monoxide is added in such an amount that the initial activity of the catalyst is reduced.

The duration of the treatment depends, among others, on the gas flow rate of the gaseous stream comprising carbon monoxide, the carbon monoxide content, the type of fixed bed, the size of the fixed bed, the amount of carbides desired, and whether all or only a part of the catalyst bed should be treated. The carbon monoxide may, for example, be provided for a few seconds, for 1 minute, for 10 minutes, for more than one hour, up to 20 hours, or even longer.

The amount of cobalt carbides formed preferably is 1 to 75 mol%, based on the total amount of cobalt atoms present in the fixed bed of catalyst, more preferably 5 to 60 mol%, even more preferably 10 to 50 mol%, most preferably 15 to 35 mol%.

The amount of carbon monoxide necessary to form carbides depends on the amount of catalyst wherein cobalt has to be converted into cobalt carbide and the cobalt atom content of such catalyst. Generally, 1 mole of carbon monoxide corresponds to 1 mole of cobalt atoms, which together will form 0.5 mole of Co₂C and 0.5 mole of CO₂. Nevertheless, it may be helpful to provide an excess of carbon monoxide to obtain sufficient cobalt carbide formation. In some cases the total amount of carbon monoxide present in the gas flow that passes the catalyst may sum up to 2 times, 5 times, 10 times, or even 100 times the amount of carbon monoxide required in the chemical reaction with the cobalt atoms.

In a preferred embodiment, the carbon monoxide content in the gaseous stream comprising carbon monoxide is increased over time during the contacting with the fixed bed. For example, one may start with passing pure inert over the catalyst particles and then gradually adding more carbon monoxide to the gaseous stream. The carbon monoxide content in the gaseous stream that is passed over the catalyst may be increased up to 0.5 mol% carbon monoxide, 1 mol% carbon monoxide, 10 mol% carbon monoxide or even 100 mol% carbon monoxide. The initial carbon monoxide content, the rate of increasing the carbon monoxide content and the final carbon monoxide content may, among others, depend on the gas flow rate of the gaseous stream comprising carbon monoxide, the type of fixed bed, the size of the fixed bed, whether or not multiple tubes with a fixed bed inside are treated at the same time, the amount of carbides desired, and the desired part of the catalyst to be treated. Preferably, the carbon monoxide content is reduced after a while, or is replaced with inert only, to achieve an activity gradient in the fixed bed of catalyst, i.e. with more catalyst deactivation in the upstream part than in the downstream part of the fixed bed.

Without wishing to be bound by any theory, the method of the present invention seems to allow the carbon monoxide to chemically bond to the metallic cobalt to form cobalt carbides.

Preferably, 90% or less of the catalyst in the fixed bed is treated with carbon monoxide, more preferably 65% or less, even more preferably 50% or less, whereby mainly catalyst located in the upstream part of the fixed bed is treated. A partial treatment of the fixed bed may be obtained by limiting the amount of carbon monoxide and/or the duration of the flow of the gaseous stream comprising carbon monoxide over the fixed bed.

One may perform the process of the present invention on a single reactor tube comprising a fixed bed of Fischer-Tropsch catalyst particles. Alternatively, more than one reactor tubes, each comprising a fixed bed of Fischer-Tropsch catalyst, may be treated at the same time.

The reduced catalyst is preferably contacted with the gaseous stream comprising carbon monoxide at a temperature in the range of from 150 to 290 °C, more preferably of from 160 to 250 °C. The reduced catalyst is may be contacted with the gaseous stream comprising carbon monoxide at any suitable pressure, preferably at a pressure at which such stream is available, for example at a pressure in the range of from 1 to 80 bar, more preferably of from 2 to 60 bar, even more preferably of from 5 to 20 bar (absolute).

It has been found that the inhibiting effect on intrinsic catalyst activity caused by the treatment with carbon monoxide can be reversed by subjecting the catalyst to hydrogen. By subjecting the catalyst to hydrogen, at least part of the cobalt carbide formed in step (b) is reduced into metallic cobalt. Therefore, if during operation of Fischer-Tropsch hydrocarbon synthesis step (c), the overall catalyst activity has been decreased below a critical level, typically below such level that the reaction temperature is to be set at an undesirably high value in order to maintain the desired reactor productivity, it is preferred to reduce the catalyst by providing a hydrogen-containing gas to the fixed bed of catalyst at a reduction temperature and pressure. Once the catalyst has been reduced, optionally after a further oxidation and reduction step, Fischer-Tropsch hydrocarbon synthesis may be resumed by supplying a gaseous feed stream comprising carbon monoxide and hydrogen in a ratio in the range of from 0.5:1 to 2:1 to the reactor at a Fischer-Tropsch reaction temperature and pressure.

Preferably therefore, if in the method according to the invention part of the catalyst in the fixed bed is provided with carbide, the method further comprises the following sequential steps after step (c):
d) contacting the fixed bed of catalyst with a hydrogen-containing gas at a reduction temperature in the range of from 180 to 350 °C and a reduction pressure in the range of from 1 to 80 bar (absolute) to obtained reduced regenerated catalyst; and
e) supplying a gaseous feed stream comprising carbon monoxide and hydrogen in a ratio in the range of from 0.5:1 to 2:1 to the reactor comprising reduced regenerated catalyst and converting carbon monoxide and hydrogen supplied with the gaseous feed stream to the reactor into hydrocarbons at a reaction temperature and reaction pressure.

Preferably, the hydrogen-containing gas comprises at least 5 vol% hydrogen, more preferably at least 10 vol%. The hydrogen-containing gas may comprise carbon monoxide in a concentration of at most 1 vol%, preferably at most 0.5 vol%.

Optionally, process steps are added between Fischer-Tropsch synthesis step (c) and the subjection of the fixed bed to reduction step (d). For example, Fischer-Tropsch product may be removed from the reactor before the catalyst is reduced. Also between reduction step (d) and Fischer-Tropsch synthesis step (e) process steps may be added. The fixed bed with reduced catalyst obtained in step (d) may be subjected to an oxidation step and a further reduction step before Fischer-Tropsch hydrocarbon synthesis is resumed in step (e).

Such oxidation step is known in the art and typically comprises treating the catalyst with an oxygen-containing gas at a temperature above 580 °C, preferably above 600 °C, and below 670 °C, preferably below 650 °C, at a pressure above 1 bar (absolute), preferably above 2 bar (absolute), and below 60 bar (absolute), preferably below 10 bar (absolute).

In another preferred embodiment of the invention, part of the catalyst in the fixed bed is provided with graphite by converting at least part of the cobalt carbide formed as hereinabove described into graphite by heating the catalyst comprising cobalt carbide in an inert gas to a temperature in the range of from 300 to 400 °C, preferably of from 320 to 380 °C. Preferably all cobalt carbide is converted into graphite. Any suitable inert gas may be used, for example nitrogen or desulphurised natural gas. The inert gas preferably is essentially free of molecular oxygen, more preferably does not comprise molecular oxygen. The inert gas may be supplied to the fixed bad at any suitable gas flow velocity. The heating is preferably carried out during 0.1 to 4 hours.

After providing part of the catalyst with graphite, the fixed bed of catalyst is cooled down to a suitable Fischer-Tropsch reaction temperature and step (c) is carried out.

It has been found that the inhibiting effect on intrinsic catalyst activity caused by the provision of the part of the catalyst with graphite can be reversed by subjecting the catalyst bed to a so-called reduction-oxidation-reduction (ROR) treatment. Therefore, if during operation of Fischer-Tropsch hydrocarbon synthesis step (c), the overall catalyst activity has been decreased below a critical level, typically below such level that the reaction temperature is to be set an undesirably high value in order to maintain the desired reactor productivity, it is preferred to subject the catalyst to an ROR treatment to obtain a reduced catalyst without graphite. After the ROR treatment, Fischer-Tropsch hydrocarbon synthesis may be resumed by supplying a gaseous feed stream comprising carbon monoxide and hydrogen in a ratio in the range of from 0.5:1 to 2:1 to the reactor at a Fischer-Tropsch reaction temperature and pressure.

Preferably therefore, if in the method according to the invention part of the catalyst in the fixed bed is provided with graphite, the method further comprises the following sequential steps after step (c):
(f) a first reduction step wherein the fixed bed of catalyst is contacted with a hydrogen-containing gas at a first reduction temperature and pressure;
(g) an oxidation step wherein the fixed bed of catalyst is contacted with an oxygen-containing gas at an oxidation temperature;
(h) a second reduction step wherein the fixed bed of catalyst is contacted with a hydrogen-containing gas at a second reduction temperature and pressure; and
(i) supplying a gaseous feed stream comprising carbon monoxide and hydrogen in a ratio in the range of from 0.5:1 to 2:1 to the reactor and converting carbon monoxide and hydrogen supplied with the gaseous feed stream to the reactor into hydrocarbons at a reaction temperature and pressure.

For steps (f) to (h) any suitable ROR conditions known in the art may be used. The first reduction temperature and pressure in step (f) are preferably the same as the second reduction temperature and pressure in step (h). The oxidation temperature may be any suitable oxidation temperature, preferably a temperature in the range of from 250 to 300 °C. The oxygen-containing gas in step (g) is preferably air.

In another embodiment of the invention, part of the catalyst in the fixed bed is provided with coke in order to deactivate part of the catalyst in step (b). Provision with coke may for example be achieved by contacting the fixed bed of reduced catalyst with a gaseous stream comprising hydrocarbons, such as for example olefins, under conditions of temperature and pressure under which coke is formed. The gaseous stream preferably is essentially free of molecular oxygen, more preferably does not comprise molecular oxygen. The gaseous stream comprising hydrocarbons may for example be an off gas stream from a Fischer-Tropsch hydrocarbon synthesis reactor. Such off gas stream typically comprises a large amount of inert compounds such as carbon dioxide and nitrogen, an amount of C1 to C5 hydrocarbons and unconverted carbon monoxide and hydrogen. Typically such stream comprises in the range of from 5 to 50 vol% C1 to C5 hydrocarbons

Conditions under which hydrocarbons are converted into coke are known in the art. The gaseous stream may be contacted with the catalyst under any suitable conditions known in the art, for example a temperature in the range of from 180 to 350 °C, preferably of from 220 to 300 °C. The pressure is preferably in the range of from 2 to 60 bar (absolute).

It has been found that by using the method according to the invention, catalyst stability is improved in the sense that it takes longer until the activity of the catalyst has decreased to such a low level that rejuvenation of the catalyst is needed.

A Fischer-Tropsch process typically comprises more than one reactors. In a Fischer-Tropsch process with several reactors, the method according to the invention may be applied in only one or in only a part of the reactors, i.e. any reactor loaded with fresh catalyst or with rejuvenated catalyst.

The invention is illustrated by the following nonlimiting examples.

### Examples

The following example shows that the intrinsic catalyst activity of a cobalt-based Fischer-Tropsch catalyst can be reduced by treating a reduced cobalt-based Fischer-Tropsch catalyst with carbon monoxide and that such activity reduction can be revered by stripping the treated catalyst with hydrogen.

### Experiment 1

A cobalt-based Fischer-Tropsch catalyst (7 grams) was loaded in a reactor tube and reduced with hydrogen at a reduction temperature of 290 °C. The reduced catalyst was cooled to 200 °C and pressurized under nitrogen to 40 bar (absolute). Subsequently, a flow of carbon monoxide (15 Nl/h) was passed over the catalyst during 20 hours. Then the reactor was cooled to 160 °C and synthesis gas was supplied to the catalyst at a gas hourly space velocity of 1,750 h⁻¹ and a pressure of 40 bar (absolute). The reaction temperature was then quickly increased to 203 °C. After 70 hours runtime, the Fischer-Tropsch hydrocarbon synthesis was stopped and the catalyst was stripped with pure hydrogen during 24 hours. During these 70 runhours the reactor productivity remained constant without increasing the reaction temperature.

### Experiment 2

In a second experiment a cobalt-based Fischer-Tropsch catalyst was reduced and subjected to carbon monoxide as in Experiment 1. The catalyst was flushed with nitrogen during 24 hours and then contacted with a flow of 30 Nl/h of hydrogen during 24 hours.

The intrinsic activity of the catalyst was measured after reduction of the catalyst with hydrogen; after the catalyst had been subjected to carbon monoxide for 13 hours; after the catalyst had been stripped with hydrogen in experiment 1 (after FT hydrocarbon synthesis) and after the catalyst had been stripped with hydrogen in experiment 2 (without FT hydrocarbon synthesis). The intrinsic activity of the catalyst after reduction of the catalyst with hydrogen (initial intrinsic activity) is set at 100%.

**Table - Relative intrinsic activity**

| Treatment | Relative intrinsic activity (%) |
|---|---|
| reduction | 100 |
| reduction + CO treatment | 73 |
| reduction + CO treatment + FT synthesis + hydrogen strip | 96 |
| reduction+ CO treatment + hydrogen strip | 98 |

## Claims

1. A method for start-up and operation of a Fischer-Tropsch reactor comprising the following sequential steps:
(a) providing a reactor with a fixed bed of reduced Fischer-Tropsch catalyst comprising cobalt as catalytically active metal wherein the catalyst has an initial intrinsic activity;
(b) deactivating part of the catalyst by providing part of the catalyst with a form of carbon;
(c) supplying a gaseous feed stream comprising carbon monoxide and hydrogen in a ratio in the range of from 0.5:1 to 2:1 to the reactor and converting carbon monoxide and hydrogen supplied with the gaseous feed stream to the reactor into hydrocarbons at a reaction temperature and reaction pressure.

2. A method according to claim 1, wherein in step (c) carbon monoxide and hydrogen are converted into hydrocarbons at a set reactor productivity and wherein the reaction temperature is initially at least 200 °C, and the set reactor productivity is maintained by adjusting the reaction temperature.

3. A method according to claim 2, wherein the reaction temperature in step (c) is initially at least 203 °C, preferably in the range of from 205 to 220 °C, more preferably in the range of from 210 to 215 °C.

4. A method according to claim any one of the preceding claims, wherein after step (b) the fixed bed consists of an upstream part and a downstream part, wherein a larger part of catalyst is deactivated in the upstream part than in the downstream part.

5. A method according to claim 4, wherein in step (b) the catalyst is deactivated such that the catalyst in the upstream part of the fixed bed has an intrinsic activity that is less than 70% of the initial intrinsic activity and the catalyst in the downstream part of the fixed bed has an intrinsic activity that is at least 70% of the initial intrinsic activity.

6. A method according to claim 4 or 5, wherein the upstream part has a length in the range of from 10 to 90% of the total length of the fixed bed, preferably of from 15 to 50% of the total length of the fixed bed.

7. A method according to any one of the preceding claims, wherein the catalyst provided in step (a) is a fresh catalyst.

8. A method according to any one of the preceding claims, wherein the catalyst does not comprise a noble metal.

9. A method according to any one of the preceding claims, wherein in step (b) part of the catalyst is provided with carbide by contacting the reduced catalyst with a gaseous stream comprising carbon monoxide, wherein the gaseous stream comprises carbon monoxide and less than 10 vol% of hydrogen, at a temperature in the range of from 150 to 290 °C, to convert at least part of the cobalt in the catalyst into cobalt carbide.

10. A method according to claim 9, wherein the temperature at which the catalyst is contacted with the gaseous stream comprising carbon monoxide is in the range of from 160 to 250 °C.

11. A method according to claim 9 or 10, further comprising the following sequential steps after step (c):
(d) contacting the fixed bed of catalyst with a hydrogen-containing gas at a reduction temperature in the range of from 180 to 350 °C and a reduction pressure in the range of from 1 to 80 bar (absolute) to obtained reduced regenerated catalyst; and
(e) supplying a gaseous feed stream comprising carbon monoxide and hydrogen in a ratio in the range of from 0.5:1 to 2:1 to the reactor and converting carbon monoxide and hydrogen supplied with the gaseous feed stream to the reactor into hydrocarbons at a reaction temperature and reaction pressure.

12. A method according to claim 9 or 10, wherein in step (b) at least part of the cobalt carbide formed is converted into graphite by heating the catalyst comprising cobalt carbide in an inert gas to a temperature in the range of from 300 to 400 °C, preferably of from 320 to 380 °C.

13. A method according to claim 12, further comprising the following sequential steps after step (c):
(f) a first reduction step wherein the fixed bed of catalyst is contacted with a hydrogen-containing gas at a first reduction temperature and pressure;
(g) an oxidation step wherein the fixed bed of catalyst is contacted with a oxygen-containing gas at an oxidation temperature;
(h) a second reduction step wherein the fixed bed of catalyst is contacted with a hydrogen-containing gas at a second reduction temperature and pressure; and
(i) supplying a gaseous feed stream comprising carbon monoxide and hydrogen in a ratio in the range of from 0.5:1 to 2:1 to the reactor and converting carbon monoxide and hydrogen supplied with the gaseous feed stream to the reactor into hydrocarbons at a reaction temperature and pressure.

14. A method according to any one of claims 1 to 8, wherein in step (b) part of the catalyst is provided with coke by contacting the reduced catalyst with a gaseous stream comprising hydrocarbons under coke-forming conditions.

15. A method according to claim 14, wherein the gaseous stream comprising hydrocarbons is off gas from a Fischer-Tropsch hydrocarbon synthesis reactor.
